# EUROPEAN PATENT SPECIFICATION

(11) **EP 4 104 761 B1**
(45) Date of publication and mention of the grant of the patent: **01.01.2025**
(21) Application number: 22177647.9
(22) Date of filing: 07.06.2022
(51) Int. Cl.: A61B 5/18, A61B 5/00

(54) **HIGH DESIGN ASSURANCE LOW COST MULTI-MODAL PILOT SAFETY SYSTEMS**
MULTIMODALE PILOTSICHERHEITSSYSTEME MIT HOHER ENTWURFSSICHERHEIT UND NIEDRIGEN KOSTEN
SYSTÈMES DE SÉCURITÉ DE PILOTE MULTIMODAL À HAUTE ASSURANCE DE CONCEPTION ET À FAIBLE COÛT

(30) Priority: 14.06.2021 US 202117347067
(43) Date of publication of application: 21.12.2022
(73) Proprietor: Rockwell Collins, Inc., Cedar Rapids, IA 52498 (US)
(72) Inventor: TIANA, Carlo L., Portland, 97215 (US); RAMADAS, Dhyuti, Portland (US)
(74) Representative: Dehns

(56) References cited:
- EP-A1- 3 459 857
- WO-A1-2015/052711
- US-A1- 2016 167 672

## Description

### TECHNICAL FIELD

The disclosure relates to pilot safety systems.

### BACKGROUND

Prior art pilot safety systems are disclosed in WO2015/052711, EP3459857 and US2016/167672.

### SUMMARY

The invention is defined in claim 1. Preferred embodiments are defined in dependent claims 2-9.

### BRIEF DESCRIPTION OF DRAWINGS

The numerous advantages of the disclosure may be better understood by those skilled in the art by reference to the accompanying figures in which:
- FIG. 1: is a flow diagram of a pilot-monitoring safety system, in accordance with one or more embodiments of the present invention.
- FIG. 2: is a conceptual image of a head worn display, in accordance with one or more embodiments of the present invention.
- FIG. 3: is a graph showing heart rate data collected under increasingly challenging flight conditions, in accordance with one or more embodiments of the present invention.
- FIG. 4: is a plot showing eye gaze data and associated heat maps collected under increasingly challenging flight conditions, in accordance with one or more embodiments of the present invention.

### DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. In the following detailed description of embodiments of the present disclosure, numerous specific details are set forth in order to provide a more thorough understanding of the inventive concepts. However, it will be apparent to one of ordinary skill in the art having the benefit of the present disclosure that the inventive concepts disclosed herein may be practiced without these specific details. In other instances, well-known features may not be described in detail to avoid unnecessarily complicating the present disclosure. The inventive concepts disclosed herein are capable of other embodiments or of being practiced or carried out in various ways. Also, it is to be understood that the phraseology and terminology employed herein is for the purpose of description and should not be regarded as limiting.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present). In addition, use of the "a" or "an" are employed to describe elements and components of embodiments of the present inventive concepts. This is done merely for convenience and to give a general sense of the inventive concepts, and "a" and "an" are intended to include one or at least one and the singular also includes the plural unless it is obvious that it is meant otherwise.

Finally, as used herein any reference to "one embodiment" or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination or sub-combination of two or more such features, along with any other features which may not necessarily be expressly described or inherently present in the present disclosure.

As head worn displays proliferate in the aviation industry (e.g., for pilots), features aimed at supporting semi-autonomous single pilot operations are increasingly desirable. When an Air-Transport Category Aircraft (e.g., carrying passengers or cargo) operates with a single crew, it is of critical importance to determine the health, awareness and mental state of the crew for a continuous and safe flight. Many possible solutions exist to monitor pilot health and engagement in the cockpit. However, most solutions are bulky, expensive, complex, or subject to insurmountable certification hurdles. Yet, a high design assurance level (DAL) is critical for pilot-monitoring, since without a high DAL, safety is compromised with potentially catastrophic consequences.

Embodiments of the present disclosure advantageously combine simple, low-cost biometric sensors with a supervisory logic system that compares and combines the output of each sensor to determine a health state of the pilot (e.g., to detect whether the pilot is alert and capable of piloting the aircraft). The redundant and independent configuration of the sensors may aid in the certification of a high-criticality autopilot system that assumes control of the aircraft in response to a determination that the pilot is unresponsive. The present disclosure differs from other solutions due to low cost, simplicity, and reliability, which are key aspects in manufacturing avionics systems at a high volume.

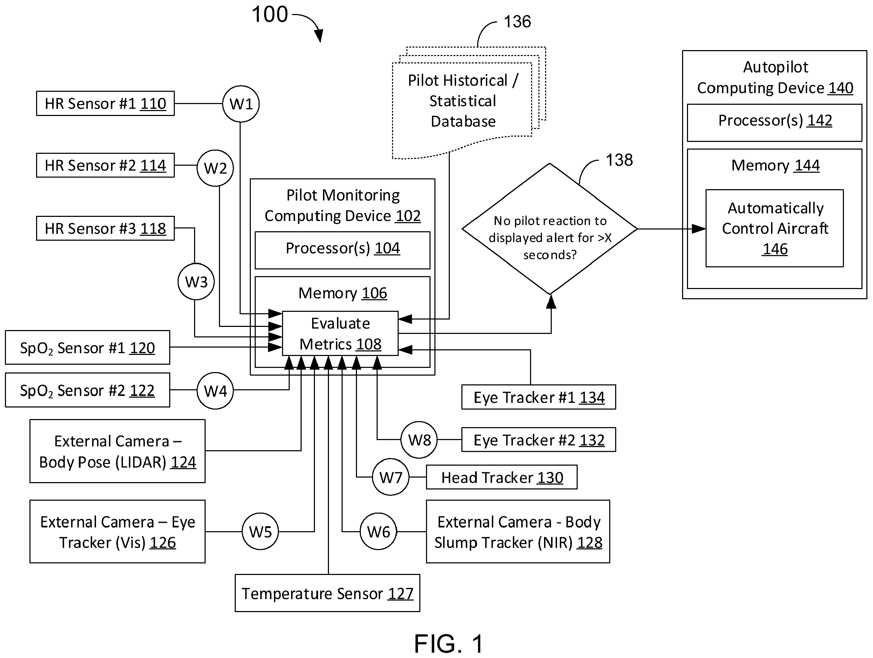

Referring now to FIG. 1, a flow diagram of a pilot-monitoring safety system 100 is shown in accordance with one or more embodiments of the present disclosure. The pilot-monitoring safety system 100 may include at least a pilot-monitoring computing device 102 and an autopilot computing device 140. In some embodiments, the computing devices 102 and 140 may be part of an avionics computing system on-board an aircraft (not shown). The computing devices 102 and 140 may each be a controller (e.g., computer) respectively including one or more processors 104, 142 and a memory 106, 144. For the purposes of the present disclosure, the term "processor" or "processing element" may be broadly defined to encompass any device having one or more processing or logic elements, for example, one or more central processing units (CPUs), one or more graphics processing units (GPUs), one or more micro-processor devices, one or more application specific integrated circuit (ASIC) devices, one or more field programmable gate arrays (FPGAs), or one or more digital signal processors (DSPs). In this sense, the one or more processors may include any device configured to execute algorithms and/or instructions (e.g., program instructions stored in memory), and may be configured to perform the method steps described in the present disclosure. The memory medium may include any storage medium known in the art suitable for storing program instructions executable by the associated one or more processors. For example, the memory medium may include, but is not limited to, a read-only memory (ROM), a random-access memory (RAM), a magnetic or optical memory device (e.g., hard disk), a magnetic tape, a solid-state drive and the like.

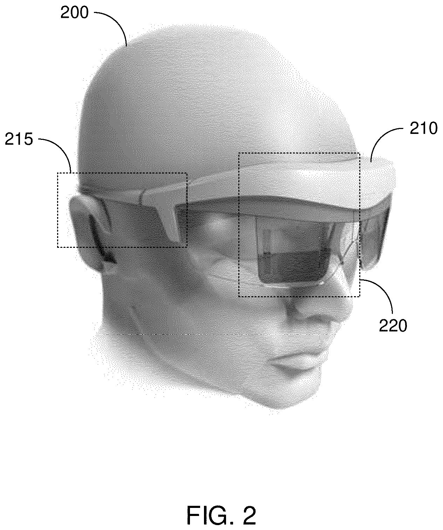

Referring now to FIG. 2, a conceptual image of a user 200 (e.g., pilot) of the pilot-monitoring safety system 100 is shown. The pilot-monitoring safety system 100 includes a head worn display 210. The head worn display 210 may be, for example, a visor, however the present disclosure is not limited thereto. For example, the head worn display 210 may comprise goggles, glasses, a helmet, etc. In some embodiments, the head worn display 210 is a virtual reality (VR), augmented reality (AR), or mixed reality (MR) system. The head worn display 210 may include an opaque, transparent, or semitransparent display. In some embodiments, the pilot-monitoring computing device 102 may be on-board or embodied in the head worn display 210 (e.g., part of the same mainboard or motherboard, and/or part of the same housing).

Referring back to FIG. 1, the pilot-monitoring safety system 100 includes one or more heart rate sensors (e.g., heart rate monitors). For example, the system 100 may include a heart rate sensor 110, a heart rate sensor 114, and a heart rate sensor 118. The heart rate sensors 110, 114, and 118 may employ an optical method to measure heart rate such as photoplethysmography (PPG), or alternatively, an electrical method such as electrocardiography (ECG). Either method may generate similar basic heart rate measurements (e.g., using fully automated algorithms to measure heart rate, such as the Pan-Tompkins algorithm). ECG sensors measure the bio-potential generated by electrical signals that control the expansion and contraction of heart chambers, while PPG sensors use a light-based technology to measure the blood volume controlled by the heart's pumping action. PPG sensors may measure heart rate by shining light from an LED through the skin (e.g., of the pilot 200) and measuring the scattering of the light from blood vessels. In some embodiments, in addition to measuring the heart rate, the heart rate sensors may measure blood oxygen saturation (SpO2). The heart rate sensors may be, for example, the POLAR H10, EMPATICA E4, WELLUE O2RING, or any sensor including similar fundamental components.

The heart rate sensors 110, 114, and 118 may be configured to generate separate heart rate measurements of the user 200 of the head worn display 210. For example, the heart rate sensor 110 may be configured to generate primary heart rate measurements, the heart rate sensor 114 may be configured to generate secondary heart rate measurements, and the heart rate sensor 118 may be configured to generate tertiary heart rate measurements. In this way, each of the heart rate sensors 110, 114, and 118 generate separate measurements, which increases the resiliency and DAL of the system 100. In the case of a failure of one of the heart rate sensors (for example, by generating inaccurate measurements, or no measurements at all), the others of the heart rate sensors may still generate accurate measurements.

According to the invention, the one or more heart rate sensors 110, 114, and 118 are attached, secured, or affixed to the head worn display 210. For example, referring again to FIG. 2, the heart rate sensors 110, 114, and 118 may be attached, secured, or affixed to a temple portion 215 of the head worn display 210 (e.g., attached at an inner side of the head worn display 210 such that the heart rate sensors are adjacent to the skin of the pilot 200). Situating the heart rate sensors 110, 114, and 118 close to the skin of the pilot 200 may increase the accuracy of the heart rate measurements. Additionally, incorporating the heart rate sensors 110, 114, and 118 in the head worn display 210 as a cohesive package reduces complexity by avoiding the use of chest straps or wrist straps, which are cumbersome and unrealistic for the pilot 200 to wear during flight. Additional heart rate or other biometric sensors may be attached to one or more surfaces of the aircraft cockpit. The additional sensors may operate symbiotically with sensors attached to the head worn display 210 for increased reliability.

The pilot-monitoring safety system 100 includes one or more blood oxygen saturation sensors 120 and 122 (e.g., pulse oximeters). The oxygen saturation sensors 120 and 122 may employ transmissive pulse oximetry and may be placed on a thin part of the skin of the user 200 (e.g., fingertip, earlobe, foot, etc.). The oxygen saturation sensors 120 and 122 may be configured to transmit two wavelengths of light through the skin to a photodetector. By measuring the changing absorbance at each of the wavelengths, the absorbances due to the pulsing arterial blood are determined. Alternatively, the sensors 120 and 122 may employ reflectance pulse oximetry which does not require a thin section of the skin of the user 200. The blood oxygen saturation sensors may be, for example, the WELLUE O2RING, or any sensor including similar fundamental components.

Like the other biometric sensors, the blood oxygen saturation sensors 120 and 122 may be configured to generate separate blood oxygen saturation measurements of the user 200 of the head worn display 210. For example, the sensor 120 may be configured to generate primary blood oxygen saturation measurements, and the sensor 122 may be configured to generate secondary blood oxygen saturation measurements. In this way, each of the sensors 120 and 122 generate separate measurements, and, in the case of a failure of one of the blood oxygen saturation sensors, the others of the sensors may still generate accurate measurements. For similar reasons as the heart rate sensors 110, 114, and 118, the blood oxygen saturation sensors 120 and 122 may be attached, secured, or affixed to the head worn display 210 (e.g., at the temple portion 215 of the head worn display 210).

The pilot-monitoring safety system 100 includes one or more body-pose cameras configured to generate body-pose measurements of the pilot 200. According to the invention, the body-pose cameras include a laser imaging, detection, and ranging (LIDAR) sensor 124 and a near infrared (NIR) or long-wave infrared (LWIR) sensor 128. The LIDAR sensor 124 may determine ranges (variable distances) by targeting the pilot 200 with a laser and measuring the time for the reflected light to return to the receiver, and construct a depth map of the pilot 200 (e.g., 3D image). The NIR or LWIR sensors 128 may detect infrared wavelengths (i.e., the heat radiating from the body of the user 200).

The LIDAR sensor 124 and IR sensor 128 may each measure the body-pose of the pilot 200 from the front or side of the pilot 200, and may detect a static pose from the pilot 200 (i.e., lack of movement), or a slumped body pose, or a tilted neck, of the pilot 200. The LIDAR sensor 124 may be, for example, an INTEL REALSENSE depth camera, or any sensor including similar fundamental components. The LIDAR sensor 124 and IR sensor 128 are each secured, attached, or affixed to one or more surfaces of the cockpit of the aircraft (external to the pilot 200 and the head worn display 210). The LIDAR sensor 124 and IR sensor 128 may be independently mounted on the surfaces of the cockpit, or may be part of a sensor package that includes other biometric sensors disclosed herein.

The pilot-monitoring safety system 100 includes one or more temperature sensors 127 configured to generate temperature measurements of the user 200 of the head worn display 210. The temperature sensor 127 may comprise a skin temperature thermometer, for example, the TEMPTRAQ wireless temperature monitor, MAXIM INTEGRATED digital thermometer, or any sensor including similar fundamental components.

The pilot-monitoring safety system 100 includes a plurality of eye trackers 126, 132, and 134 configured to generate eye-gaze measurements of the user of the head worn display 210. The one or more eye trackers 126, 132 and 134 may comprise one or more image sensors 213 (i.e., cameras) configured to capture images of the eyes of the pilot 200, and thus track movement of the eyes over time. The eye trackers 126, 132, and 134 may comprise a complementary metal oxide semiconductor (CMOS) sensor, a charge-coupled device (CCD) sensor, etc. The eye trackers 126, 132, and 134 may be configured to detect visible light, infrared, ultraviolet, or a combination (e.g., multispectral fusion sensors). The eye trackers 132 and 134 may be, for example, a TOBII PRO 2 eye tracker, or any eye tracker including similar fundamental components or including similar functionality.

One eye tracker 126 is placed externally from the pilot 200 and the head worn display 210 (e.g., attached, affixed, or secured, to one or more surfaces of the cockpit of the aircraft). On the other hand, other eye trackers 132 and 134 are attached, secured, or affixed to the head worn display 210. For example, referring again to FIG. 2, the eye trackers 132 and 134 may be attached, secured, or affixed to a rim portion 220 of the head worn display 210 (e.g., attached at an inner side of the head worn display 210 so that the eye trackers are adjacent and in front of the eyes of the pilot 200). The physical separation of the eye tracker 126 (attached to a surface of the cockpit) from the eye trackers 132 and 134 (attached to the head worn display 210) may provide redundant eye-gaze measurements, and thus may increase the resilience and DAL of the system 100.

The pilot-monitoring safety system 100 includes one or more head position trackers 130 configured to generate head positions measurements of the user 200 of the head worn display 210. The head position tracker 130 may measure the user's head motion using a combination of accelerometer(s), gyroscope(s), magnetometer(s), global positioning system (GPS) sensors, altimeters, etc. The head position tracker 130 may generate measurements based on the translation and rotation of the user (e.g., in six degrees of freedom). In some embodiments, the head position tracker 130 may be one or more inertial measurement units (IMUs). In other embodiments, the head position tracker 130 may comprise one or more IR trackers or cameras near the user. In general, the head position tracker 130 may be any device that measures the orientation and position of the head of the user of the HWD 210 over time.

It is noted that the sensors 110, 114, 118, 120, 122, 124, 126, 127, 128, 130, 132, and 134 may be communicatively coupled to the pilot monitoring computing device 102 using a wired or wireless connection. In some embodiments, the sensors are wirelessly connected using a WiFi or Bluetooth connection. In some embodiments, the sensors are connected by wire to the pilot monitoring computing device 102 to reduce latency (for example, if the computing device 102 is part of the head worn display 210).

The measurements generated by the sensors 110, 114, 118, 120, 122, 124, 126, 127, 128, 130, 132, and 134 may be recorded and stored in a historical or statistical database 136. The database 136 may collect data from a plurality of users of the system 100 (e.g., a population of hundreds or thousands of pilots over a long period of time). The data stored in the database 136 may be used to determine a correlation of each separate measurement (heart rate, eye gaze, body pose, temperature, head position) with others of the measurements with respect to a health state of the pilot 200. The health state may be quantitative (e.g., a numerical value) and/or qualitative (e.g., "pilot under stressful conditions," "pilot partially incapacitated," "pilot fully incapacitated").

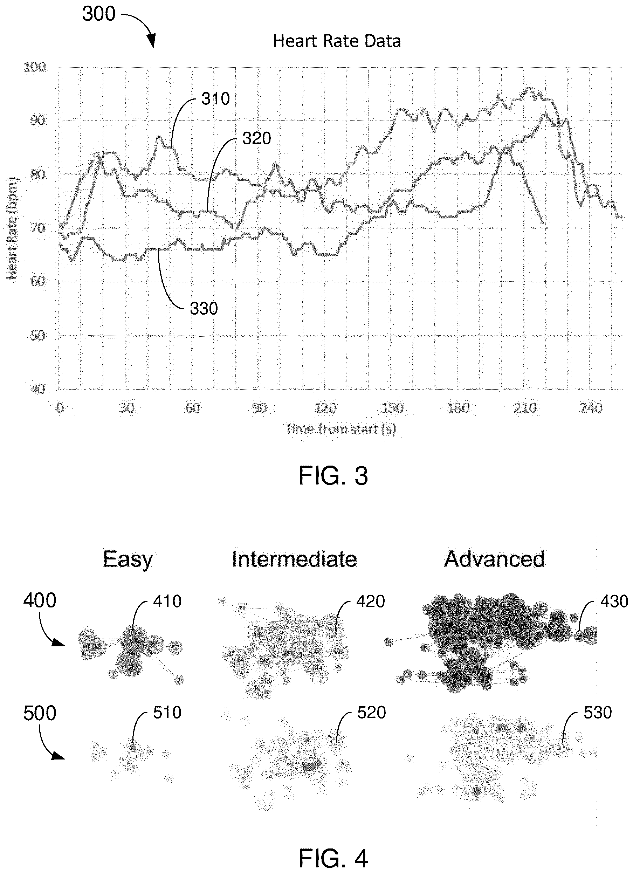

FIG. 3 is a graph 300 showing heart rate measurement data collected from a pilot under increasingly challenging conditions of a flight simulation, in accordance with one or more embodiments of the present disclosure. The horizontal axis represents time (seconds) and the vertical axis represents heart rate (beats per minute). The line 310 represents data collected under the advanced difficulty setting, the line 320 represents data collected under the intermediate difficulty setting, and the line 330 represents data collected under the easy difficulty setting. Predictably, the advanced difficulty setting resulted in the highest average heart rate of the user over the simulation (line 310), while the easy difficulty setting resulted in the lowest average heart rate of the user (line 330).

FIG. 4 shows plots 400 of eye-gaze measurement data collected under the increasingly challenging flight conditions of the simulation described with respect to FIG. 3, and heat maps 500 associated with the plots 400. The plots 400 represent the movement of the user's eyes to different instruments, gauges, and displays of an aircraft cockpit. The plot 410 and associated heat map 510 represent data collected under the easy difficulty setting, the plot 420 and associated heat map 520 represent data collected under the intermediate difficulty setting, and the plot 430 and associated heat map 530 represent data collected under the advanced difficulty setting. Predictably, the advanced difficulty setting resulted in the greatest dispersion of the eye gaze (plot 430 and heat map 530), while the easy difficulty setting resulted in the least dispersion of the eye gaze (plot 410 and heat map 530).

FIGS. 3 and 4 illustrate the concept of correlating different biometric sensor traces to construct a health state of a pilot of an aircraft. Low stress flight simulator conditions (e.g., the easy difficulty setting with a reduced workload) result in lower heart rates and less dispersion of eye gaze, while relatively high stress flight simulator conditions result in higher heart rates and more dispersion of eye gaze. Collecting data from hundreds or thousands of users of the system 100 (where each user's state is measured by a variety of biometric sensors) may produce a robust model to determine the health state of the user. Data collected from pilot populations over time (such as the data presented in FIGS. 3-4) may be classified against pilot performance in general, or against specific pilot tasks (takeoff, hazard avoidance, landing, for example), and stored in the database 136. The data collected from pilot populations over time may then be compared to the metrics collected in real-time by the sensors and head and eye trackers described herein.

Referring back to FIG. 1, after generating measurements (heart rate, eye gaze, body pose, temperature, and/or head position) using the sensors 110, 114, 118, 120, 122, 124, 126, 127, 128, 130, 132, and/or 134, the measurements are evaluated 108, and the health state of the user 200 is determined. The health state of the user 200 may be quantitative (e.g., a numerical value calculated based on numerical measurement values such as beats per minute, blood oxygen saturation, etc.), and/or qualitative (e.g., "pilot under stressful conditions," "pilot partially incapacitated," "pilot fully incapacitated").

For example, each of the measurements (heart rate, eye gaze, body pose, temperature, and/or head position) may be normalized (such that each measurement value ranges from 0 to 1, e.g., 0.0, 0.3, 0.7, 1.0, etc.). In some embodiments, each type of measurement may be averaged (for example, the heart rate measurements collected by the sensors 110, 114, and 118 may be averaged into an average heart rate). In some embodiments, the normalized and/or averaged values may then be used to determine an overall or net health state value (for example, by adding or multiplying the values).

Additionally or alternatively, qualitative labels may be assigned to each measurement value based on passed thresholds. For example, if the user exceeds an average measured heart rate of 150 bpm (e.g., normalized to 0.75), the contribution of the heart rate measurements to the health state may be labeled as "user under stressful conditions." Alternatively, if the user has an averaged measured heart rate below 15 bpm, or even 0 bpm (e.g., no heart beat or flatline), the contribution of the heart rate measurements to the health state may be labeled "user partially incapacitated" or "user fully incapacitated." The qualitative labels may indicate a dangerous condition of the user 200 (which may require the engagement of the autopilot computing device 140 to land the aircraft to avoid catastrophe). If all of the different measurements indicate a particular dangerous condition, then the health state of the user 200 may be associated with the particular dangerous condition.

According to the invention, the contribution of the measurements (heart rate, eye gaze, body pose, temperature, and/or head position) to the determination of the health state of the user 200 is weighted based on a-priori knowledge. In some embodiments, the measurements from the different sensors may be assigned a weight (relative value) based on data stored in the historical or statistical database 136, and the system 100 may employ weighted voting. For example, if the measurements from heart rate sensor 110 agree (i.e., correlate closely) with the measurements from heart rate sensor 114, but not with the measurements from the heart rate sensor 118, the measurements from heart rate sensor 118 may be excluded from contributing to the determination of the health state of the user. Alternatively, the measurements from the heart rate sensor 118 may be given a lower weight (lower reliability) and thus lower contribution to the determination of the health state of the user.

If the health state of the user 200 indicates a dangerous condition, then a visual alert may be presented to the user 200 using the head worn display 210. A user may then respond to the alert, for example, using a user input device (mouse, keyboard, touchscreen, etc.). Alternatively and/or additionally, the alert may be audial and output from a headset or speakers to the user 200, and the user 200 may respond to the alert using a microphone. Alternatively and/or additionally, the alert may be haptic and generated by a vibrating mechanical device (e.g., an eccentric rotating mass [ERM] actuator, a linear resonant actuator [LRA], or a piezoelectric actuator) incorporated into the head mounted display 210, or other surfaces in contact with the pilot's body, such as the seat or the yoke. The user 200 may dismiss the alert in the case of a false positive (i.e., the user 200 being safe and alert), or based on the judgment of the user 200. However, if the user 200 does not respond 138 to the alert after a set amount of time has elapsed (e.g., 30 seconds), the pilot monitoring computing device 102 transmits a signal to the autopilot computing device 140 to automatically control the aircraft. For example, the autopilot computing device 140 may be communicatively coupled to a flight management system (FMS) and one or more flight control surfaces (ailerons, rudders, elevators, spoilers, flaps, etc.). The autopilot computing device 140 may guide the aircraft to land at a nearby runway (or on a flat plain, or open ocean), thus avoiding catastrophic consequences in the event that the user 200 is impaired.

It is believed that the present disclosure and many of its attendant advantages will be understood by the foregoing description, and it will be apparent that various changes may be made in the form, construction, and arrangement of the components without departing from the disclosed subject matter or without sacrificing all of its material advantages. The form described is merely explanatory, and it is the intention of the following claims to encompass and include such changes. Furthermore, it is to be understood that the invention is defined by the appended claims.

## Claims

1. A pilot-monitoring safety system (100), comprising:
a head worn display (210);
one or more heart rate sensors (110, 114, 118) configured to generate heart rate measurements of a user of the head worn display, wherein the one or more heart rate sensors are attached, secured, or affixed to the head worn display (210);
one or more eye trackers (126, 132, 134) configured to generate eye-gaze measurements of the user of the head worn display, wherein the one or more eye trackers (132, 134) are attached, secured, or affixed to the head worn display;
one or more blood oxygen saturation sensors (120, 122) configured to generate blood oxygen saturation measurements of the user, wherein the blood oxygen saturation sensors (120, 122) are attached, secured, or affixed to the head worn display;
a LIDAR sensor (124) and IR sensor (128) each configured to generate body-pose measurements of the user, wherein the LIDAR sensor and IR sensor are attached, secured or affixed to one or more surfaces of a cockpit of an aircraft;
one or more secondary eye trackers (126) configured to generate secondary eye-gaze measurements of the user, wherein the one or more secondary eye trackers are secured, attached or affixed to one or more surfaces of a cockpit of an aircraft;
a temperature sensor (127) configured to generate temperature measurements of the user of the head worn display;
one or more head position trackers (130) configured to generate head position measurements of the user of the head worn display, wherein the one or more head position trackers are secured, attached or affixed to one or more surfaces of a cockpit of an aircraft;
a pilot-monitoring computing device (102) configured to:
determine a health state of the user based on the heart rate measurements, the eye-gaze measurements, the blood oxygen saturation measurements, the body-pose measurements, the temperature sensor measurements, and the head position measurements;
wherein a contribution of the heart rate measurements, the eye-gaze measurements,
the body-pose measurements, the temperature measurements and the head position measurments to the determination of the health state of the user is weighted based on a-priori knowledge;
wherein the pilot-monitoring safety system (100) employs weighted voting; and
responsive to the health state indicating a dangerous condition of the user, present an alert to the user using the head worn display; and
an autopilot computing device configured to:
responsive to a predetermined time period elapsing without a response to the alert from the user, automatically control an aircraft;

2. The system of claim 1, wherein the alert is a visual alert presented on at least one of a display of the head worn display, or a display of the cockpit of the aircraft.

3. The system of claim 1 or 2, wherein the alert is an audial alert output by at least one of speakers or headphones.

4. The system of any preceding claim, wherein the alert is a haptic alert generated by a vibrating mechanical device secured, attached, or affixed to the head worn display.

5. The system of any preceding claim, wherein the heart rate measurements being greater than a heart rate threshold indicates the dangerous condition of the user.

6. The system of any preceding claim, wherein the heart rate measurements being less than a heart rate threshold indicates the dangerous condition of the user.

7. The system of any preceding claim, wherein automatically controlling the aircraft comprises directing the aircraft to land using one or more flight control surfaces of the aircraft.

8. The system of claim 1, wherein the one or more heart rate sensors comprise three heart rate sensors, wherein a first the- heart rate sensor (110) is configured to generate primary heart rate measurements, a second heart rate sensor (114) is configured to generate secondary heart rate measurements, and a third heart rate sensor (118) is configured to generate tertiary heart rate measurements.

9. The system of claim 1, wherein the one or more blood oxygen saturation sensors comprise two oxygen saturation sensors, wherein a first oxygen saturation sensor (120) is configured to generate primary blood oxygen saturation measurements and a second oxygen saturation sensor (122) is configured to generate secondary blood oxygen saturation measurements.

## Patentansprüche

1. Pilotüberwachungssicherheitssystem (100), umfassend:
eine am Kopf getragene Anzeige (210);
einen oder mehrere Herzfrequenzsensoren (110, 114, 118), die dazu konfiguriert sind, Herzfrequenzmessungen eines Benutzers der am Kopf getragenen Anzeige zu erzeugen, wobei der eine oder die mehreren Herzfrequenzsensoren an der am Kopf getragenen Anzeige (210) angebracht, gesichert oder befestigt sind;
einen oder mehrere Eyetracker (126, 132, 134), die dazu konfiguriert sind, Blickmessungen des Benutzers der am Kopf getragenen Anzeige zu erzeugen, wobei der eine oder die mehreren Eyetracker (132, 134) an der am Kopf getragenen Anzeige angebracht, gesichert oder befestigt sind;
einen oder mehrere Blutsauerstoffsättigungssensoren (120, 122), die dazu konfiguriert sind, Blutsauerstoffsättigungsmessungen des Benutzers zu erzeugen, wobei die Blutsauerstoffsättigungssensoren (120, 122) an der am Kopf getragenen Anzeige angebracht, gesichert oder befestigt sind;
einen LIDAR-Sensor (124) und einen IR-Sensor (128), die jeweils dazu konfiguriert sind, Körperhaltungsmessungen des Benutzers zu erzeugen, wobei der LIDAR-Sensor und der IR-Sensor an einer oder mehreren Oberflächen eines Cockpits eines Flugzeugs angebracht, gesichert oder befestigt sind;
einen oder mehrere sekundäre Eyetracker (126), die dazu konfiguriert sind, sekundäre Blickmessungen des Benutzers zu erzeugen, wobei der eine oder die mehreren sekundären Eyetracker an einer oder mehreren Oberflächen eines Cockpits eines Flugzeugs gesichert, angebracht oder befestigt sind;
einen Temperatursensor (127), der dazu konfiguriert ist, Temperaturmessungen des Benutzers der am Kopf getragenen Anzeige zu erzeugen;
einen oder mehrere Kopfpositionstracker (130), die dazu konfiguriert sind, Kopfpositionsmessungen des Benutzers der am Kopf getragenen Anzeige zu erzeugen, wobei der eine oder die mehreren Kopfpositionstracker an einer oder mehreren Oberflächen eines Cockpits eines Flugzeugs gesichert, angebracht oder befestigt sind;
eine Pilotüberwachungsrechenvorrichtung (102), die zu Folgendem konfiguriert ist:
Bestimmen eines Gesundheitszustands des Benutzers basierend auf den Herzfrequenzmessungen, den Blickmessungen, den Blutsauerstoffsättigungsmessungen, den Körperhaltungsmessungen, den Temperatursensormessungen und den Kopfpositionsmessungen;
wobei ein Beitrag der Herzfrequenzmessungen, der Blickmessungen, der Körperhaltungsmessungen, der Temperaturmessungen und der Kopfpositionsmessungen zur Bestimmung des Gesundheitszustands des Benutzers basierend auf apriorischem Wissen gewichtet wird;
wobei das Pilotüberwachungssicherheitssystem (100) eine gewichtete Abstimmung einsetzt; und
als Reaktion darauf, dass der Gesundheitszustand einen gefährlichen Zustand des Benutzers angibt, Präsentieren einer Warnung an den Benutzer unter Verwendung der am Kopf getragenen Anzeige; und
eine Autopilotrechenvorrichtung, die zu Folgendem konfiguriert ist:
als Reaktion darauf, dass ein vorgegebener Zeitraum ohne eine Reaktion von dem Benutzer auf die Warnung abläuft, automatisches Steuern des Flugzeugs.

2. System nach Anspruch 1, wobei die Warnung eine visuelle Warnung ist, die auf mindestens einer von einer Anzeige der am Kopf getragenen Anzeige oder einer Anzeige des Cockpits des Flugzeugs präsentiert wird.

3. System nach Anspruch 1 oder 2, wobei die Warnung eine akustische Warnung ist, die über mindestens eines von Lautsprechern und/oder Kopfhörern ausgegeben wird.

4. System nach einem der vorhergehenden Ansprüche, wobei die Warnung eine haptische Warnung ist, die durch eine vibrierende mechanische Vorrichtung erzeugt wird, die an der am Kopf getragenen Anzeige gesichert, angebracht oder befestigt ist.

5. System nach einem der vorhergehenden Ansprüche, wobei, wenn die Herzfrequenzmessungen über einem Herzfrequenzschwellenwert liegen, dies den gefährlichen Zustand des Benutzers angibt.

6. System nach einem der vorhergehenden Ansprüche, wobei, wenn die Herzfrequenzmessungen unter einem Herzfrequenzschwellenwert liegen, dies den gefährlichen Zustand des Benutzers angibt.

7. System nach einem der vorhergehenden Ansprüche, wobei das automatische Steuern des Flugzeugs Anweisen des Flugzeugs zum Landen unter Verwendung einer oder mehrerer Flugsteuerungsflächen des Flugzeugs umfasst.

8. System nach Anspruch 1, wobei der eine oder die mehreren Herzfrequenzsensoren drei Herzfrequenzsensoren umfassen, wobei ein erster Herzfrequenzsensor (110) dazu konfiguriert ist, primäre Herzfrequenzmessungen zu erzeugen, ein zweiter Herzfrequenzsensor (114) dazu konfiguriert ist, sekundäre Herzfrequenzmessungen zu erzeugen, und ein dritter Herzfrequenzsensor (118) dazu konfiguriert ist, tertiäre Herzfrequenzmessungen zu erzeugen.

9. System nach Anspruch 1, wobei der eine oder die mehreren Blutsauerstoffsättigungssensoren zwei Sauerstoffsättigungssensoren umfassen, wobei ein erster Sauerstoffsättigungssensor (120) dazu konfiguriert ist, primäre Blutsauerstoffsättigungsmessungen zu erzeugen, und ein zweiter Sauerstoffsättigungssensor (122) dazu konfiguriert ist, sekundäre Blutsauerstoffsättigungsmessungen zu erzeugen.

## Revendications

1. Système de sécurité de surveillance de pilote (100), comprenant :
un affichage porté sur la tête (210) ;
un ou plusieurs capteurs de fréquence cardiaque (110, 114, 118) configurés pour générer des mesures de fréquence cardiaque d'un utilisateur de l'affichage porté sur la tête, dans lequel les un ou plusieurs capteurs de fréquence cardiaque sont attachés, fixés ou collés à l'affichage porté sur la tête (210) ;
un ou plusieurs dispositifs de suivi oculaire (126, 132, 134) configurés pour générer des mesures de suivi du regard de l'utilisateur de l'affichage porté sur la tête, dans lequel les un ou plusieurs dispositifs de suivi oculaire (132, 134) sont attachés, fixés ou collés à l'affichage porté sur la tête ;
un ou plusieurs capteurs de saturation en oxygène du sang (120, 122) configurés pour générer des mesures de saturation en oxygène du sang de l'utilisateur, dans lequel les capteurs de saturation en oxygène du sang (120, 122) sont attachés, fixés ou collés à l'affichage porté sur la tête ;
un capteur LIDAR (124) et un capteur IR (128), chacun configuré pour générer des mesures de posture du corps de l'utilisateur, dans lequel le capteur LIDAR et le capteur IR sont attachés, fixés ou collés à une ou plusieurs surfaces d'un cockpit d'un aéronef ;
un ou plusieurs dispositifs de suivi oculaire secondaires (126) configurés pour générer des mesures de suivi du regard secondaires de l'utilisateur, dans lequel les un ou plusieurs dispositifs de suivi oculaire secondaires sont fixés, attachés ou collés à une ou plusieurs surfaces d'un cockpit d'un aéronef ;
un capteur de température (127) configuré pour générer des mesures de température de l'utilisateur de l'affichage porté sur la tête ;
un ou plusieurs dispositifs de suivi de position de tête (130) configurés pour générer des mesures de position de tête de l'utilisateur de l'affichage porté sur la tête, dans lequel les un ou plusieurs dispositifs de suivi de position de tête sont fixés, attachés ou collés à une ou plusieurs surfaces d'un cockpit d'un aéronef ;
un dispositif informatique de surveillance de pilote (102) configuré pour :
déterminer l'état de santé de l'utilisateur sur la base des mesures de fréquence cardiaque, des mesures de suivi du regard, des mesures de saturation en oxygène du sang, des mesures de posture du corps, des mesures de capteur de température et des mesures de position de la tête ;
dans lequel une contribution des mesures de fréquence cardiaque, des mesures de suivi du regard, des mesures de posture du corps, des mesures de température et des mesures de position de la tête à la détermination de l'état de santé de l'utilisateur est pondérée sur la base d'une connaissance a priori ;
dans lequel le système de sécurité de surveillance de pilote (100) utilise un vote pondéré ; et
en réponse à l'état de santé indiquant un état dangereux de l'utilisateur, présenter une alerte à l'utilisateur à l'aide de l'affichage porté sur la tête ; et
un dispositif informatique de pilotage automatique configuré pour :
en réponse à une période de temps prédéterminée s'écoulant sans réponse à l'alerte de la part de l'utilisateur, commander automatiquement un aéronef .

2. Système selon la revendication 1, dans lequel l'alerte est une alerte visuelle présentée sur au moins un affichage de l'affichage porté sur la tête ou sur un affichage du cockpit de l'aéronef.

3. Système selon la revendication 1 ou 2, dans lequel l'alerte est une alerte sonore émise par au moins l'un d'un haut-parleur ou d'un casque.

4. Système selon une quelconque revendication précédente, dans lequel l'alerte est une alerte haptique générée par un dispositif mécanique vibrant fixé, attaché ou collé à l'affichage porté sur la tête.

5. Système selon une quelconque revendication précédente, dans lequel les mesures de fréquence cardiaque supérieures à un seuil de fréquence cardiaque indiquent l'état dangereux de l'utilisateur.

6. Système selon une quelconque revendication précédente, dans lequel les mesures de fréquence cardiaque inférieures à un seuil de fréquence cardiaque indiquent l'état dangereux de l'utilisateur.

7. Système selon une quelconque revendication précédente, dans lequel la commande automatique de l'aéronef comprend le fait de diriger l'aéronef pour atterrir à l'aide d'une ou de plusieurs surfaces de commande de vol de l'aéronef.

8. Système selon la revendication 1, dans lequel les un ou plusieurs capteurs de fréquence cardiaque comprennent trois capteurs de fréquence cardiaque, dans lequel un premier capteur de fréquence cardiaque (110) est configuré pour générer des mesures de fréquence cardiaque primaires, un deuxième capteur de fréquence cardiaque (114) est configuré pour générer des mesures de fréquence cardiaque secondaires et un troisième capteur de fréquence cardiaque (118) est configuré pour générer des mesures de fréquence cardiaque tertiaires.

9. Système selon la revendication 1, dans lequel les un ou plusieurs capteurs de saturation en oxygène du sang comprennent deux capteurs de saturation en oxygène, dans lequel un premier capteur de saturation en oxygène (120) est configuré pour générer des mesures de saturation en oxygène du sang primaires et un second capteur de saturation en oxygène (122) est configuré pour générer des mesures de saturation en oxygène du sang secondaires.
